Europäisches Patentamt

European Patent Office

Office européen des brevets

(19)

(11) Publication number: **0 492 214 A2**

(12) **EUROPEAN PATENT APPLICATION**

(21) Application number: **91120951.8**

(22) Date of filing: **06.12.91**

(51) Int. Cl.5: **C12N 15/12**, C07K 13/00, C12N 15/63, C12P 21/02, A61K 37/02, C12P 21/08

(30) Priority: **27.12.90 EP 90811030**
**30.04.91 EP 91810327**

(43) Date of publication of application:
**01.07.92 Bulletin 92/27**

(84) Designated Contracting States:
**AT BE CH DE DK ES FR GB GR IT LI LU MC NL SE**

(71) Applicant: **F. HOFFMANN-LA ROCHE AG**
**Postfach 3255**
**CH-4002 Basel(CH)**

(72) Inventor: **Devos, René**
**56 Edith Cavellstraat**
**B-8400 Oostende(BE)**
Inventor: **Fiers, Walter**
**3 Beukendreef**
**B-9070 Destelbergen(BE)**
Inventor: **Plaetinck, Geert**
**767 Dendermondsesteenweg**
**B-9070 Destelbergen(BE)**
Inventor: **Tavernier, Jan**
**2 Bottelweg**
**B-9860 Balegem(BE)**
Inventor: **van der Heyden, José**
**23 Zink**
**B-9820 Munte(BE)**

(74) Representative: **Mezger, Wolfgang, Dr. et al**
**Grenzacherstrasse 124 Postfach 3255**
**CH-4002 Basel(CH)**

(54) **Human Interleukin-5 receptor.**

(57) It is an object of the present invention to provide a recombinant α-chain of the human Interleukin 5-receptor or parts thereof, DNA-sequences coding for such a receptor or for parts thereof, host cells transformed with such vectors and a process for the production of such α-chains of the human Interleukin 5-receptor employing such transformed host cells and pharmaceutical compositions containing such receptors or fragments thereof and their use for the treatment of illnesses, e.g. chronic asthma.

EP 0 492 214 A2

Interleukin-5 (IL-5 or IL5) is a lymphokine secreted by T cells and mastcells having biological activities on B cells and eosinophils. The activity on B cells seems to be restricted to the murine system. No detectable activity can be found in a panel of human B-cell activation or differentiation assays. [Clutterbuck et al., Eur. J. Immunol. 17, 1743-1750 (1987)].

In murine hematopoiesis, IL-5 is a selective signal for the proliferation and differentiation of the eosinophilic lineage [Yamaguchi et al., J. Exp. Med. 167, 43-56 (1988)]. In this respect, IL-5 function shows analogies with colony-stimulating factors for other myeloid lineages. Also, human (h) IL-5 is very potent in the activation of human eosinophils [Lopez et al., J. Exp. Med. 167, 219-224 (1988); Saito et al., Proc. Natl. Acad. Sci USA 85, 2288-2292)].

Interleukin 5 mediates its activity through a cell membrane receptor-complex. This complex has been characterized physicochemically in both the murine and human system. Mouse pre B cell lines depending on IL5 for their growth have been developed from bone marrow and are used for IL5-receptor analysis [Rolink et al., J. Exp. Med. 169, 1693-1701 (1989)]. The human IL5-receptor can be studied on a subclone of the promyelocytic cell line HL60 induced towards eosinophil differentiation [Plaetinck et al., J. Exp. Med. 172, 683-691 (1990)].

Eosinophilic differentiation is initiated using sodium butyrate. Only high affinity (Kd = 30 pM) IL5 binding sites can be found on these cells. However cross-linking studies reveal the presence of two polypeptide chains involved in IL5 binding, with molecular masses closely resembling the murine IL5R-$\alpha$- and -$\beta$ chains.

A soluble human IL5R$\alpha$-chain (shIL5R$\alpha$) could be used as an IL-5 antagonist in chronic asthma or other disease states with demonstrated eosinophilia. In addition the shIL5R$\alpha$ or the $\alpha$-chain itself or the whole high affinity receptor, consisting of the $\alpha$-chain and the $\beta$-chain [Tavernier et al., Cell 66, 1175-1184 (1991)] could be used as a tool for screening for IL-5 antagonists.

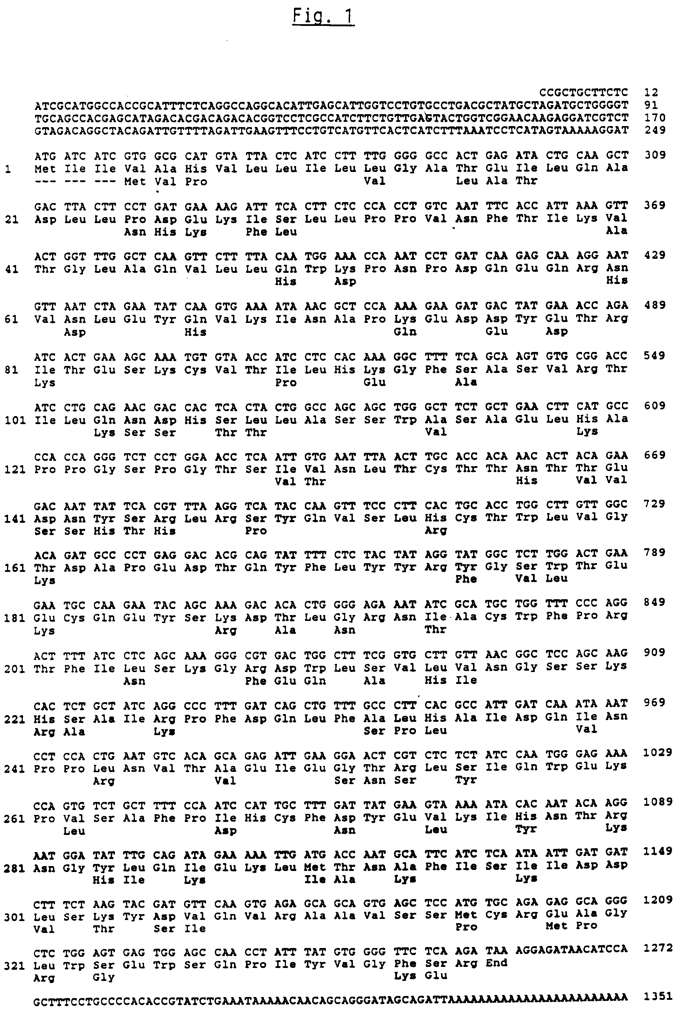

It is therefore an object of the present invention to provide a DNA sequence coding for the $\alpha$-chain of the human Interleukin-5 receptor (hIL5-R) or parts thereof especially for a shIL5R$\alpha$, a vector comprising such a DNA sequence, proor eukaryotic host cells transformed with such a vector, recombinant proteins coded for by a DNA sequence of the present invention especially recombinant shIL5R$\alpha$ whereby those comprising an amino acid sequence as shown in Figure 1 are specifically preferred and a process for the production of such proteins by cultivating a transformed host of the present invention in a suitable medium and isolating a recombinant protein of the present invention.

Preferred DNA sequences of the present invention are those comprising insert cDNAs of cDNA clones obtainable from suitable cDNA-libraries, e.g. as described in Example 4 by hybridization screening as described e.g. in Example 5. Further preferred are such insert cDNAs as prepared in Example 5, like e.g. those of cDNA clone "λgt11-hIL5R$\alpha$12". Furthermore preferred DNA sequences of the present invention are such sequences coding for a shIL5R$\alpha$, specifically such sequences, comprising a sequence as shown in Figure 1.

The cloning of a DNA sequence of the present invention can be achieved in the following manner. Murine cell lines which contain the murine IL-5-receptor (mIL5R) in membrane-bound form, can be cultivated according to methods known in the art or as specifically described in e.g. in Example 2. Such cells can then be harvested by centrifugation, lysed and a membrane extract can be prepared by using a suitable detergent, e.g. Triton-X-100. For the isolation of the $\alpha$-chain of mIL5R, the membrane extract, cleared by centrifugation, can be passed over an immuneaffinity matrix. The corresponding antibodies for such an immunematrix namely the ones to the $\alpha$-chain of the mIL5R can be prepared and coupled to an appropriate matrix by methods well known in the art or as specifically described e.g. in Examples 1-3. The $\alpha$-chain of the mIL5-R can be further purified by sodium dodecylsulfate polyacrylamide gelelectrophoresis (SDS-PAGE) and blotted to an appropriate matrix.

The thus-purified murine IL-5-receptor chain can be characterized by methods of peptide chemistry which are known in the state of the art, such as, for example, N-terminal amino acid sequencing or enzymatic as well as chemical peptide cleavage. Fragments obtained by enzymatic or chemical cleavage can be separated according to usual methods such as, for example, HPLC and can themselves be subjected to further N-terminal sequencing.

Starting from the so-obtained amino acid sequence information oligonucleotides can be produced according to methods known in the state of the art [see e.g. Sambrook et al., s.a.] taking into consideration the degeneration of the genetic code.

cDNA or genomic DNA libraries can be produced according to methods known in the art [Sambrook et al., "Molecular Cloning", 2nd. ed., Cold Spring Harobor Laboratory Press (1989)], whereby cDNA libraries on the basis of an mRNA-preparation from cell lines expressing with or without induction murine or human IL5R, e.g. as specifically described in Example 4, are preferred. Such libraries can then be screened by oligonucleotides [Sambrook et al., s.a.]. Once a specific clone has been identified in such a manner the phage harboring the desired DNA sequence of the invention can be isolated [Sambrook et al., s.a.] and the corresponding inserts characterized by restriction enzyme cleavage pattern analysis or sequencing according to standard procedures (Sambrook et al., s.a.). It is understood that also DNA sequences hybridizing under stringent hybridization conditions (see e.g. Sambrook et al.,; s.a.) to those of the present invention and coding for proteins which still bind IL5 and such proteins itself are an object of the present invention. Such DNA sequences can be prepared e.g. by mutagenesis methods known in the art (see e.g. Sambrook et al.) starting from the corresponding natural DNA sequences.

On the basis of the thus-determined sequences and of the already known sequences for certain receptors, those partial sequences which code for a soluble receptor subunit can be determined and cut out from the complete sequence using known methods [Sambrook et al., s.a., see also Maliszewski and Fanslow, Tibtech., 8, 324-329 (1990)] in case that a specific insert cDNA of a clone of the present invention does not already code for such a shIL5Rα as this is for example the case for cDNA clone "λgt11-hIL5Rα12".

The complete sequence or such partial sequences can then be integrated using known methods into vectors described in the state of the art for their amplification and/or expression in prokaryotes [Sambrook et al., s.a.]. Suitable prokaryotic host organisms are, for example, gram-negative and gram-positive bacteria such as, for example, E. coli strains such as E. coli HB 101 [ATCC No. 33 694] or E. coli W3110 [ATCC No. 27 325] and E.coli MC1061 [Casadabam and Cohen, J. Mol. Biol. 138, 179-207 (1980)], the latter two already harboring plasmid "p3" (Sambrook et al., s.a.) in case that pCDM8-type vectors like πVX or pshIL5Rα (see Example 9) will be amplified or B. subtilis strains.

Furthermore, nucleic acid sequences in accordance with the invention can be integrated using known methods into suitable vectors for expression in eukaryotic host cells, such as, for example, yeast, insect cells and mammalian cells. Such vectors as those mentioned above are also an object of the present invention.

A typical expression vector for mammalian cells contains an efficient promoter element in order to produce a good transcription rate, the DNA sequence to be expressed and signals for an efficient termination and polyadenylation of the transcript. Additional elements which can be used are "enhancers" which lead to again intensified transcription and sequences which e.g. can bring about a longer half life of the mRNA. For the expression of nucleic acid sequences in which the endogenous sequence fragment coding for a signal peptide is missing, there can be used vectors which contain such suitable sequences which code for signal peptides of other known proteins. See, for example, the vector pLJ268 described by Cullen, B.R. in Cell 46, 973-982 (1986) as well as Sharma, S. et al. in "Current Communications in Molecular Biology", edt. by Gething, M.J., Cold Spring Harbor Lab. (1985), pages 73-78.

Most of these vectors which are used for a transient expression of a particular DNA sequence in mammalian cells contain the replication origin of the SV40 virus. In cells which express the T-antigen of the virus (e.g. COS cells), these vectors are reproduced abundantly. A transient expression as described e.g. in Example 10 is, however, not limited to COS cells. In principle any transfectable mammalian cell line can be used for this purpose. Signals which can bring about a strong transcription are e.g. the early and late promoters of SV40, the promoter and enhancer of the "major immediate-early" gene of HCMV (human cytomegalovirus), the LTR's ("long terminal repeats") of retroviruses such as, for example, RSV, HIV and MMTV. There can, however, also be used signals of cellular genes such as e.g. the promoters of the actin and collagenase genes.

Alternatively, however, stable cell lines which have the specific DNA sequence integrated into the genome (chromosome) can also be obtained. For this, the DNA sequence is cotransfected together with a selectable marker, e.g. neomycin, hygromycin, dihydrofolate reductase (dhfr) or hypoxanthin guanine phosphoribosyl transferase (hgpt). The DNA sequence stably incorporated in the chromosome can also be amplified abundantly. A suitable selection marker for this is, for example, dihydrofolate reductase (dhfr). Mammalian cells (e.g. CHO cells), which contain no intact dhfr gene, are thereby incubated with increasing amounts of methotrexate after transfection has been effected. In this manner cell lines which contain more than a thousand copies of the desired DNA sequence can be obtained.

Mammalian cells which can be used for the expression are e.g. cells of the human cell lines Hela [ATCC CCL2] and 293 [ATCC CRL 1573] as well as 3T3 [ATCC CCL 163] and L cells, e.g. [ATCC CCL 149], (CHO) cells [ATCC CCL 61], BHK [ATCC CCL 10] cells as well as the CV 1 [ATCC CCL 70] and the COS cell lines [ATCC CRL 1650, CRL 1651].

Suitable expression vectors include, for example, vectors such as pBC12MI [ATCC 67 109], pSV2dhfr [ATCC 37 146], pSVL [Pharmacia, Uppsala, Sweden], pRSVcat [ATCC 37 152], pMSG [Pharmacia, Uppsala, Sweden] and pCDM8 type plasmids like e.g. pshIL5Rα [see Example 7] which has been deposited transformed in E.coli MC1061 (harboring plasmid p3) under the conditions of the Budapest Treaty for patent purposes at the Deutsche Sammlung von Mikroorganismen und Zellkulturen GmbH (DSM) in Braunschweig, Federal Republic of Germany on April 17, 1991 under accession number DSM 6479 The plasmid pshIL5Rα can be isolated from the deposited transformed E.coli as known in the art and described e.g. in detail in Example 9. Prokaryotic host cells transformed with such plasmids comprising a DNA sequence of the present invention, especially transformed E.coli cells are also an object of the present invention as well as such plasmids used for their transformation.

The manner in which these cells are transfected depends on the chosen expression system and vector system. An overview of these methods is to be found e.g. in Pollard et al., "DNA Transformation of Mammalian Cells" in "Methods in Molecular Biology" [Nucleic Acids Vol. 2, 1984, Walker, J.M., ed, Humana, Clifton, New Jersey]. Further methods are to be found in Chen and Okayama ["High-Efficiency Transformation of Mammalian Cells by Plasmid DNA", Molecular and Cell Biology 7, 2745-2752, 1987] and in Felgner [Felgner et al., "Lipofectin: A highly efficient, lipid-mediated DNA-transfection procedure", Proc. Nat. Acad. Sci. USA 84, 7413-7417, 1989]. Eukaryotic host cells transfected with a suitable plasmid (vector) of the invention, especially mammalian host cells, whereby COS-cells are specifically preferred, as well as the plasmids used for their transfection and expression of the corresponding recombinant protein, are also an object of the present invention.

The baculovirus expression system, which has already been used successfully for the expression of a series of proteins (for an overview see Luckow and Summers, Bio/Technology 6, 47-55, 1988), can be used for the expression in insect cells. Recombinant proteins can be produced in authentic form or as fusion proteins. The thus-produced proteins can also be modified such as, for example, glycosylated (Smith et al., Proc. Nat. Acad. Sci. USA 82, 8404-8408, 1987). For the production of a recombinant baculovirus which expresses the desired protein there is used a so-called "transfer vector". Under this there is to be understood a plasmid which contains the heterologous DNA sequence under the control of a strong promoter, e.g. that of the polyhedron gene, whereby this is surrounded on both sides by viral sequences. The transfer vector is then transfected into the insect cells together with DNA of the wild type baculovirus. The recombinant viruses which result in the cells by homologous recombination can then be identified and isolated according to known methods. An overview of the baculovirus expression system and the methods used therein is to be found in Luckow and Summers, "A Manual of Methods for Baculovirus Vectors and Insect Cell Culture Procedures", Texas Agricultural Experimental Station, Texas A & M University, Bulletin No. 1555, 2nd edition, 1988.

The expressed IL-5-receptor chain or fragments thereof can then be purified from the cell mass or the culture supernatants according to methods of protein chemistry which are known in the state of the art, such as, for example, precipitation e.g. with ammonium sulfate, dialysis, ultrafiltration, gelfiltration, ion-exchange chromatography, SDS-PAGE, isoelectric focusing, affinity chromatography like immunoaffinity chromatography, HPLC or the like.

Moreover, the IL-5-receptor chain or fragments thereof in accordance with the invention can be used for the detection of IL-5 agonists or antagonists according to procedures which are known in the state of the art.

The IL-5-receptor chain or the fragments thereof of the present invention as well as their physiologically compatible salts, which can be manufactured according to methods which are known in the state of the art, can also be used for the treatment of illnesses in which IL-5 is involved in their course and/or the production of corresponding pharmaceutical preparations. For this purpose, one or more of the said compounds, where desired or required in combination with other pharmaceutically active substances, can be processed in a known manner with the usually used solid or liquid carrier materials. The dosage of such preparations can be effected having regard to the usual criteria in analogy to already used preparations of similar activity and structure. Such pharmaceutical preparations and the use of the compounds of the present invention for therapeutical purposes are also an object of the present invention.

After the invention has been described in general hereinbefore, the following Figures and Examples are intended to illustrate details of the invention, without thereby limiting it in any manner.

Figure 1

Nucleic acid sequence of shIL5Rα and deduced amino acid sequence whereby the corresponding amino acid sequence of the mIL5Rα is indicated below by showing only such amino acids which are different from the ones of the human sequence. Sequences are represented by standard abbreviations for nucleotides and amino acids.

Figure 2

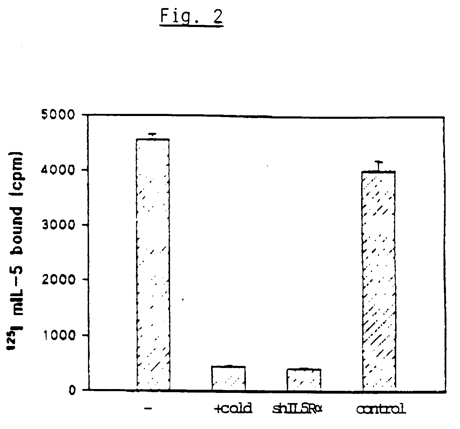

Figure 2 gives a characterization of shIL5Rα. Any details are given in Example 11.

Figure 3

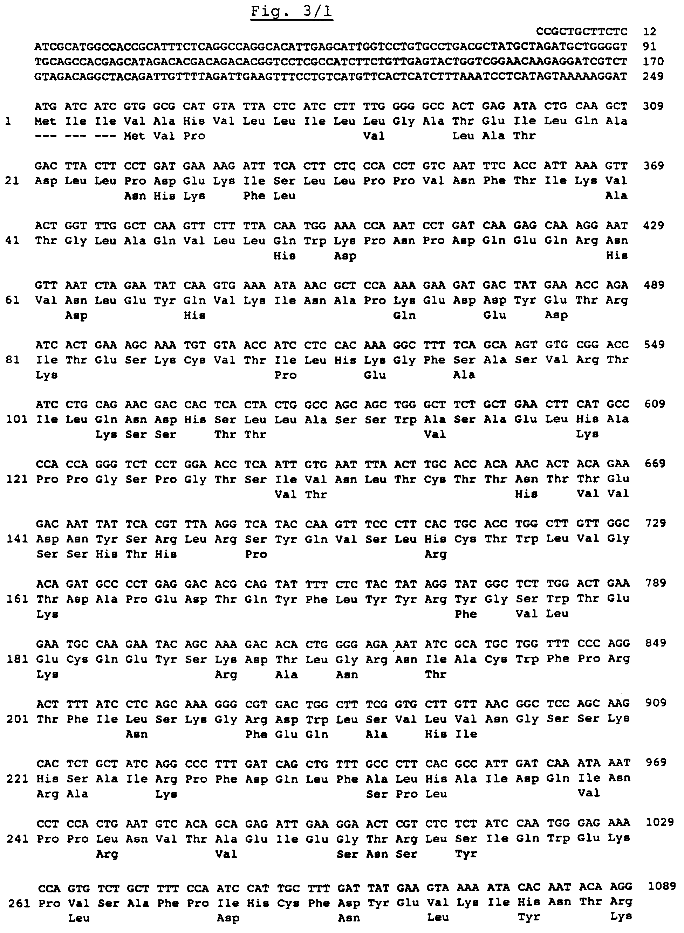

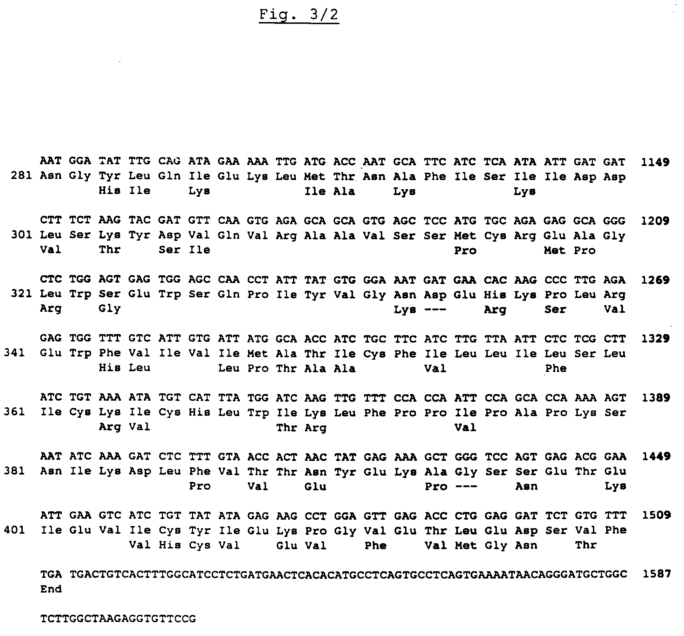

Figure 3 shows the DNA-sequence and derived amino acid sequence of hIL5Rα whereby the corresponding amino acid sequence of the mIL5Rα is indicated below by showing only such amino acids which are different from the ones of the human sequence. Sequences are represented by standard abbreviations.

Example 1

Production of monoclonal antibodies against the murine IL5R

Immunization was carried out basically as described by A. Rolink et al. (s.a.). Briefly: at day 0, $2X10^7$ B13 cells [Rolink et al., s.a.] were washed with phosphate buffered saline (PBS-A), mixed with complete Freund's adjuvant (CFA) and injected into the hind footpath of Wistar rats. This was repeated without Freund's adjuvant (FA) on day 5 and 7. On day 8, regional lymph nodes were removed and a cell suspension was prepared. These cells were fused using PEG 1500 (Boehringer) with Sp2/0-Ag14 cells [ATCC CRL 1581] at a ratio of 5:1,5. Cells were plated in microtiterplates in the presence of 500 pg/ml of recombinant hIL-6 [Haegeman et al., Europ. J. Biochem. 159, 625-632 (1986)]. The next day, the same volume of medium containing a 2x conc. of aminopterin was added for selection of hybrid cells. Cells were refed at day 8 with medium without aminopterin. Hybridomas were selected on the ability of their supernatant to inhibit the mIL5 [Tavernier, J. et al., DNA 8, 491-501 (1989)] or an mouse interleukin-3 (mIL3) driven proliferation of B13 cells (measured by a $^3$Hdeoxy-cytidin incorporation assay as known in the art). Conditioned medium from WEHI-3 cells (ATCC No. TIB68) was used as a source of mIL3. Supernatants demonstrating inhibiting activity were retested in a competition-binding assay with radiolabeled [according to methods known in the art] mIL5 or "R52" [a monoclonal antibody recognizing only the $\beta$-chain of the IL-5-R (Rolink et al. s.a.)] on B13 cells. Monoclonal antibodies directed only to the $\alpha$-chain of the mIL-5-R were identified on their ability to inhibit almost completely mIL5 binding and by immunprecipitation of the corresponding mIL-5-R chain. Selected hybridomas were recloned by the limiting dilution method.

Example 2

Immunoaffinity purification of the mIL5R-$\beta$-chain

B13 cells were grown in large spinner flasks in Iscove's modified Dulbecco's medium (Gibco Laboratories, grand Island N.Y., USA) containing 5% fetal calf serum, 2mM L-glutamine, $50\mu$g/ml gentamycin, and 100 units/ml recombinant mouse IL-5, to a density of $2 \times 10^6$ cells/ml. Cells from 10 l cultures were concentrated by centrifugation, washed with PBS and lysed in 200ml PBS containing 1% Triton-X-100 and a cocktail of protease inhibitors (1mM PMSF, 10mM benzamidine.HCl, 100 U/ml aprotinin). After 10 min on ice, the lysate was centrifuged for 10 min at 1000 x g and cleared by ultracentrifugation (100.000 x g) for 90 min at 4°C. The supernatant was diluted with NaCl to a final concentration of 0.5 M, and used for purification. "R52" was covalently bound to protein G-Sepharose 4 Fast Flow (Pharmacia, LKB Biotechnology AB, Uppsala, Sweden) according to Schneider et al.[J. Biol. Chem. 257, 10766 (1982)], at a concentration of 5 mg/ml gel. Two hundred ml lysate of B13 cells was passed at 4C° over 2 ml protein G-Sepharose 4 Fast Flow followed by 2ml R52-linked protein G-Sepharose 4 Fast Flow both packed in a 1cm diameter column. The flow through was then reloaded on both columns. The gel was washed extensively (100ml) with a buffer containing 50mM Tris-HCl (pH8.2), 1mM EDTA, 0.5M NaCl, 0.5% NP40, followed by 10 ml 0.1% (NP40). Next, the retained proteins were eluted in 4ml 50mM diethylamine (pH11) containing 0.1% Nonidet P40 (NP40), neutralized by addition of 1M $NaH_2PO_4$ and concentrated by lyophilization. The purity was assessed by SDS-PAGE and Coomassie staining of 2.5% of the eluate.

Example 3

Immunoaffinity purification of the murine IL5R $\alpha$-chain

B13 cell lysates from 2 x 10^10 cells (run through fractions of the "R52"-immunoaffinity column used to purify the β-chain doublet according to Example 2) were mixed overnight by 4°C with 2ml hydrazide avidgel AX (Bioprobe Int. Inc.) armed with 10 mg mAbs recognizing the mIL5R α-chain. The gel was then poored into a column, and after extensive washing (50mM Tris.HCl, pH 8.2, 1mM EDTA, 0.5M NaCl, 0.5% NP40; followed by 0.1% NP40 in $H_2O$) elution was performed using 50mM diethylamine, pH11, 0.01% NP40. Selected fractions were immediately lyophilized and resuspended in 2x Laemmli buffer, in the presence of β-mercaptoethanol. Samples were run through a 1.5 mm 10% PAGE-SDS gel. The gel was fixed in 10% HAc, 30% methanol and stained with Coomassie Brilliant Blue. Slices containing the 60 kDa mIL5R α-chain were treated with SDS buffer, sliced further and electrophoresed in a new PAGE-SDS gel.

After transfer to an Immobilon-P membrane (Millipore Corp.), and staining with amido black, the 60 kDa band was excised and in situ digested with trypsin. Peptides were separated on a C4-reversed-phase column and subjected to sequence analysis using a 470A-type gas-phase sequenator equipped with an on-line 120A-type PTH-amino acid analyser (Applied Biosystems Inc., Foster City, CA). Amino acid sequences (standard abbreviations of amino acids) and the sequences of corresponding sets of oligonucleotide probes, synthesized according to methods known in the art, are shown below:

### peptide 1

1 2 3 4 5 6 7 8 9 10 11 12
W G E W S Q P I Y V G K

### oligonucleotide-set 1 : 32-mers

```
                  T
5' CC IAC GTA AAT IGG CTG IGA CCA CTC ICC CCA  3'
          A   G       T          T
```

```
                  T
5' CC IAC GTA AAT IGG CTG ACT CCA CTC ICC CCA  3'
          A   G       T   G      T
```

### peptide 2

1 2 3 4 5 6 7 8
H V D L E Y H V

### oligonucleotide-set 2 : 23-mers

```
5' AC ATG ATA TTC TAA ATC IAC ATG 3'
       G   G   C   C   G       G
```

```
5' AC ATG ATA TTC IAG ATC IAC ATG 3'
       G   G   C       G       G
```

Example 4

6

Construction of unidirectional λGT11 cDNA libraries

1. Murine pre-B cell B13 cDNA library

mRNA was extracted from B13 cells Using the "fast-track" mRNA isolation system (Invitrogen Corp.). Using this protocol, poly(A)$^+$ mRNA was directly isolated from cell lysates using oligo(dT) cellulose; yields were around 50 $\mu$g per $10^8$ cells. 5 mg poly(A)$^+$ mRNA was reverse transcribed using an oligo dT-Not1 primer-adaptor (5'-AATTCGCGGCCGC(T)$_{15}$-3', Promega Corp.) and cloned Moloney Murine Leukemia Virus RNaseH$^-$ Reverse Transcriptase (BRL Life Technologies, Inc.). EcoR1 linkered double stranded cDNA was made using described procedures (Sambrook et al., s.a.). Not1 cleavage was used to generate a unique 3' sticky-end, and cDNAs were size selected (>1.000 bp) on a 1% agarose gel. After elution using the "gene clean" protocol (BIO 101 Inc.), cDNAs were ligated into the EcoR1-Not1 arms of the λgt11 Sfi-Not vector (Promega Corp.). After in vitro packaging around 40 x $10^6$ recombinant phages were obtained.

2. Human, HL60 clone (butyrate induced) cDNA library

Prior to mRNA purification, butyrate induced HL60 clone 15 cells [Fischkoff, Leukemia Res. 12, 679-686 (1988); Plaetinck et al., J. Exp. Med. 172, 683-691 (1990); HL60: ATCC-No. CCL 240] were checked for proper $^{125}$I-hIL5 binding (around 2000 binding sites per cell). The same protocols as for 4.1 were used, and a comparable yield of recombinant phage was obtained.

Example 5

Screening of murine and human cDNA libraries

2 sets of oligonucleotide probes "Oligonucleotide 1" and "Oligonucleotide 2", see Example 3 were used for screening under different hybridization conditions (see below), dependent on the type of probe used by methods known in the art (Sambrook et al., s.a.). Results are presented in the scheme below:

1. 2 cDNA clones (λgt11-mIL5Rα2,3) were selected from part of the murine cDNA library (1.2x$10^6$ plaques were screened), on the basis of hybridization with both sets of oligonucleotide probes. For that purpose, plaque lifts were prepared as described using Biodyne A transfer membranes (Pall), (Sambrook et al., s.a.). Oligonucleotide 1 was radioactively labeled by kinasing (Sambrook et al., s.a.) and was hybridized under "intermediate stringency" hybridization conditions (see below). Oligonucleotide 2 was radioactively labeled by kinasing (Sambrook et al., s.a.) and was hybridized under "low stringency" hybridization conditions (see below).

2. 1 cDNA clone (λgt11-hIL5Rα8) was selected from part of the human cDNA library (2.4 x $10^6$ plaques were screened), on basis of hybridization with both "oligonucleotide 1" and the cDNA insert derived by methods known in the art from the murine λgt11mIL5Rα2.

Oligonucleotide 1 was radioactively labeled by kinasing (Sambrook et al., s.a.) and was hybridized under "low stringency" hybridization conditions. The cDNA insert form λgt11mIL5Rα2 was radioactively labeled by random labeling (Sambrook et al., s.a.) and was hybridzed under "intermediate stringency" hybridization conditions.

3. 5 additional cDNA clones (λgt11-hIL5Rα11→15) were selected from half of the human cDNA library screened in 2. using the mIL5Rα2 cDNA probe. Hybridization was under "intermediate stringency" conditions.

4. 35 additional cDNA clones (λgt11-hIL5Rα16→51) were selected from the other half of the human cDNA library screened in 2. using the hIL5Rα8-cDNA probe. Hybridization was under "high stringency" conditions (see below).

Hybridization conditions

L) "low stringency" hybridization conditions:
- prehdybridization: 5 x SSC (citrate buffered salt solution known in the art, see e.g. Sambrook et al., s.a.), 5x Denhardt's, 0.1% SDS, 0.05% sodium pyrophosphate, 100 $\mu$g/ml sonicated salmon sperm DNA; overnight at 42°C.
- hybridization: prehybridization buffer was replaced by the same buffer but including the radioactively labeled probe.
- washes: 4 consecutive washes (around 30 min. each) with 2x SSC, 0.1% SDS at 37°C:

7

I) "intermediate stringency" hybridization conditions:
- prehybridization: 20% formamide, 5x SSC, 5x Denhardt's, 5mM EDTA, 25mM sodium phosphate (pH 6.5), 0.05% sodium pyrophosphate, 100 $\mu$g/ml sonicated salmon sperm DNA; overnight at 42°C.
- hybridization: prehybridization buffer was replaced by the same buffer but including the radioactively labeled probe.
- washes: 4 consecutive washes (around 30 min. each) with 2x SSC, 0.1% SDS at 37°C.

H) "high stringency" hybridization conditions:
- prehybridization: 6x SSC, 5x Denhardt's, 0.5% SDS, 100 $\mu$g.ml$^{-1}$, sonicated salmon sperm DNA, overnight at 68°C.
- hybridization: 6x SSC, 5x Denhardt's, 0.5% SDS, 5mM EDTA, 100$\mu$g.ml$^{-1}$ sonicated salmon sperm DNA including the radioactively labeled probe.
- washes: the following consecutive washes (around 30 min. each) were performed:
  - 2x SSC, 0.1% SDS at room temperature (twice).
  - 0.1x SSC, 0.1% SDS at 68°C (twice).

Example 6

Sequencing

All cDNAs were subcloned in pGEM7zf type vectors (Promega Corp.), and Exo III deletion mutants have been generated. Sequencing was performed using a protocol based on the Sanger procedure and involving Taq polymerase and single stranded DNA on an automated 37OA DNA Sequencer.

Example 7

Construction of plasmid "pshIL5R$\alpha$"

Plasmid constructions were carried out as described in the following paragraphs. In case that no specific references or details of preparation are given standard methodology according to Sambrook et al. (1989), Molecular Cloning. A Laboratory Manual (2nd edn). Cold Spring Harbor, N.Y., Cold Spring Harbor Laboratory Press, was used.

The insert from phage $\lambda$gt11-hIL5R$\alpha$12 (see Example 5) was excised using EcoR1 and Not1 restriction enzymes. Both sticky ends were filled in using E.coli DNA polymerase 1 Klenow fragment in the presence of all four deoxynucleotide triphosphates, and non-palindromic BstX1 linkers were added using T4 DNA ligase. The sequence of these linkers is as follows:

$$5' \text{ CTTTAGAGCACA } 3'$$
$$3' \text{ GAAATCTC } 5'.$$

In a next step, the modified insert was ligated into plasmid pCDM8 [Seed and Aruffo, Proc. Natl. Acad. Sci. USA, 84, 3365 (1987); Aruffo and Seed, Proc. Natl. Acad. Sci. USA, 84, 8573 (1987); Seed, Nature, 329, 840 (1987)] and the construct with the appropriate orientation versus the CMV-promoter was chosen for further analysis.

Example 8

Transformation of E.coli MC1061(p3)

Transformation of E.coli MC1061 (p3) with the plasmid pshIL5R$\alpha$ of Example 7 was achieved by the electroporation procedure. A Gene Pulser from Bio-Rad (Richmond, CA, USA) with the following settings: 25 $\mu$F, 2.5 kV and 200 Ohms was used according to the instructions of the manufacturer.

Example 9

Isolation of Plasmid DNA

Plasmid DNA from transformed E.coli MC1061 as described in Example 8 was prepared using a standard procedure [Birnboim and Doly, Nucl. Acids Res. 7, 1513 (1979); Sambrook et al., 1989, s.a.] based upon alkali lysis of the cells, followed by a cesium-chloride ultracentrifugation step. In this way plasmid pshIL5Rα was separated from plasmid p3. The insert coding for shIL5Rα was cut out of pshIL5Rα and sequenced as described in Example 6. The complete nucleic acid sequence and the deduced amino acid sequence of the shIL5Rα are shown in Figure 1.

Example 10

Expression of shIL5Rα in COS-1 cells

COS-1 cells were transfected using the DEAE-Dextran protocol as described in Sambrook et al., 1989, s.a.. Briefly, subconfluent COS-1 cells were harvested by trypsinization and replated at $2.3 \times 10^4$ cells/cm$^2$ 24 hours prior to transfection. The monolayers were washed twice with minimal essential medium (MEM)-Hepes pH 7.2 and incubated for 30 minutes with the transfection mixture [10 $\mu$g pshIL5Rα isolated as described in Example 9/ 0.5 mg DEAE-dextran ($M_r$ = $2 \times 10^6$; Pharmacia, Uppsala, Sweden)/ml MEM-Hepes, pH 7.2]. Next the cells were supplemented with 8 volumes prewarmed Dulbecco's modified Eagles medium (DMEM) containing 10% foetal calf serum (FCS) and 100 $\mu$M chloroquine diphosphate, and incubated for 4 hours at 37°C. Thereafter the medium was removed by aspiration and the monolayers were washed once with DMEM and incubated for 3 days in DMEM + 10% FCS.

Example 11

Characterization of shIL5Rα

Supernatant of COS-1 cells transfected with plasmid pshIL5Rα prepared as described in Example 10 was tested for the presence of secreted shIL5Rα in a competition binding assay as follows: COS-1 cells transfected as described in Example 10 with a plasmid comprising a cDNA coding for mIL5Rα (for amino acid sequence see Figure 1), obtained from a clone as described in Example 5 and constructed as described in Example 7, were detached by treatment with phosphate buffered saline (PBS) containing 0.5 mM EDTA and 0.02% sodium azide for 30 minutes at 37°C, resuspended at $1.5 \times 10^5$ cells per 0.3 ml binding medium (DMEM + 10% FCS + 0.02% sodium azide) and incubated with 0.8 nM $^{125}$I-mIL5 at 4°C for 1 hour in the absence (Figure 2; "-", total binding) or presence (Figure 2; "+cold", non-specific binding) of 100- fold excess unlabeled mIL5. Supernatant of COS-1 cells (80% of binding medium) transfected with pshIL5Rα (Figure 2, "shIL5Rα") was tested for its capacity to inhibit the binding of $^{125}$I-mIL5. Binding was also carried out in the presence of 80% supernatant of untransfected COS-1 cells (Figure 2, "control"). To separate cell membrane bound $^{125}$I-mIL5 from free radioactivity COS-1 cells were sedimented through a phthalate oil cushion and individual pellets were counted in a gamma counter as described [Plaetinck et al., J. Exp. Med. 172, 683-691 (1990)].

Example 12

Construction of plasmid "phIL5Ra"

cDNAs encoding hIL5Rα were isolated by a 3' extension polymerase chain reaction (PCR) strategy [Fritz et al., Nucl. Acid Res. 19, 3747 (1991)]. Because the mRNA encoding shIL5Rα is only around 1350 bases long, the PCR products derived from cDNAs encoding hIL5Rα could be size-selected. In a first step, cDNAs were synthesized by priming with a random hexamer, hooked up with a known 27 base stretch at its 5' end. Then, a PCR reaction was performed between a forward primer complementary to the shIL5Rα sequence (see Fig. 1) at position 1036-1055, and a reverse primer complementary to the tail sequence of the random hexamer. Although most reaction products were aspecific, Southern blot analysis revealed IL5Rα sequences only when mRNA from butyrate induced HL60 clone 15 cells was used. DNA fragments larger than 350 bp were purified and after cleavage with Nsi1 and Sal1 subcloned in a pUC18 vector (Pharmacia, Freiburg, BRD). hIL5Rα encoding subclones were then selected by a colony hybridization step. By doing so, DNAs corresponding to shIL5Rα were counterselected. DNA sequence analysis subsequently revealed that subclones with inserts larger than 350 bp indeed encoded hIL5Rα. Next, fullsize hIL5Rα cDNA was generated by Nsi1 - Xba1 (blunted) excision of insert, and insertion into Nsi1 - Not1 (blunted) opened pshIL5Rα vector (see Example 7). The complete DNA sequence of hIL5Rα and the derived amino acid sequence are given in Fig. 3. The DNA sequence is characterized by a switch at position 1243 of the shIL5Rα DNA-sequence, resulting in a replacement of the 3' terminal part by a new sequence encompassing the transmembrane region. The cytoplasmic domain is 58 amino acids long. A domain implicated in signal transduction processes can not be found in this short cytoplasmic tail.

**Claims**

1. A DNA sequence coding for the α-chain of the human Interleukin 5-receptor or parts thereof which parts still bind human Interleukin 5.

2. A DNA sequence according to claim 1 coding for a soluble part of the α-chain of the human Interleukin 5-receptor which soluble parts still binds human Interleukin 5.

3. A DNA sequence according to claim 1 or 2 selected from the following:
   (a) a DNA sequence as given in Figure 1 as well as their complementary strands, or those which include these sequences;
   (b) DNA sequences which hybridize with sequences defined under (a) or fragments thereof;
   (c) DNA sequences which, because of the degeneration of the genetic code, do not hybridize with sequences as defined under (a) and (b), but which code for polypeptides having exactly the same amino acid sequence.

4. A vector comprising a DNA-sequence as claimed in any one of claims 1 to 3.

5. A vector as claimed in claim 4 capable of directing expression in eukaryotic host cells.

6. A pro- or eukaryotic host cell transformed with a vector as claimed in claim 4 or 5.

7. A recombinant protein coded for by a DNA-sequence as claimed in any one of claims 1-3.

8. A recombinant protein as claimed in claim 7 for the treatment of illnesses.

9. A process for the preparation of a recombinant protein as claimed in claim 7, which process comprises cultivating a transformed host-cell as claimed in claim 6 in a suitable medium and isolating said recombinant protein.

10. A pharmaceutical composition which contains one or more compounds according to claim 7, if desired in combination with additional pharmaceutically active substances and/or non-toxic, inert, therapeutically compatible carrier materials.

11. The use of a compound according to claim 7 for the treatment of illnesses, especially chronic asthma.

**Claims for the following Contracting States : GR, ES**

1. A process for the preparation of the α-chain of the human Interleukin 5-receptor or parts thereof which parts still bind human Interleukin 5 which process comprises cultivating a host cell transformed with an expression vector comprising a DNA-sequence coding for the α-chain of the human Interleukin 5-receptor or parts thereof which parts still bind human Interleukin 5.

2. A process as claimed in claim 1 whereby the DNA-sequence codes for a soluble part of the human Interleukin 5-receptor, which soluble part still binds human Interleukin 5.

3. A process as claimed in claim 1 or 2 whereby the DNA-sequence is selected from the following:
   (a) a DNA sequence as given in Figure 1 as well as their complementary strands, or those which include these sequences;
   (b) DNA sequences which hybridize with sequences defined under (a) or fragments thereof;
   (c) DNA sequences which, because of the degeneration of the genetic code, do not hybridize with sequences as defined under (a) and (b), but which code for polypeptides having exactly the same amino acid sequence.

4. A process as claimed in any one of claims 1-3 whereby said host cell is a pro- or eukaryotic host cell.

5. A process as claimed in claim 4 whereby said eukaryotic host cell is a mammalian host cell.

6. A process as claimed in claim 5 whereby said mammalian host cell is a CHO- or COS-cell.

7. A process as claimed in any one of claims 1-6 whereby the expression vector is of the pSV2-, pRSV-, pBC12MI-, pMSG- or pCDM8-type.

8. A process for the preparation of a pharmaceutical composition which process is characterized in that a compound obtained by a process as claimed in any one of claims 1-7 and if desired, additional pharmaceutically active substances are mixed with a non-toxic, inert, therapeutically compatible carrier material and the mixture is brought into a galenical application form.

9. A pharmaceutical composition which contains one or more compounds obtained according to a process as claimed in any one of claims 1-7, if desired, in combination with additional pharmaceutically active substances and/or non-toxic, inert, therapeutically compatible carrier materials.

10. The use of a compound prepared according to a process as claimed in any one of claims 1-7 for the preparation of a pharmaceutical composition according to claim 10.

11. A compound whenever prepared according to a process as claimed in any one of claims 1-7.

12. The invention as hereinbefore described.

13. A DNA-sequence comprising a DNA-sequence coding for a compound prepared according to a process as claimed in any one of claims 1-7.

14. A vector, especially for expression in an eukaryotic host cell, such vector comprising a DNA-sequence as claimed in claim 13.

15. A host cell, especially an eukaryotic host cell transformed by a vector as claimed in claim 14.

16. A host cell as claimed in claim 15 which is a CHO- or COS-cell.

# Fig. 1

```
                                                                    CCGCTGCTTCTC    12
      ATCGCATGGCCACCGCATTTCTCAGGCCAGGCACATTGAGCATTGGTCCTGTGCCTGACGCTATGCTAGATGCTGGGGT    91
      TGCAGCCACGAGCATAGACACGACAGACACGGTCCTCGCCATCTTCTGTTGAGTACTGGTCGGAACAAGAGGATCGTCT   170
      GTAGACAGGCTACAGATTGTTTTAGATTGAAGTTTCCTGTCATGTTCACTCATCTTTAAATCCTCATAGTAAAAAGGAT   249
```

```
      ATG ATC ATC GTG GCG CAT GTA TTA CTC ATC CTT TTG GGG GCC ACT GAG ATA CTG CAA GCT   309
  1   Met Ile Ile Val Ala His Val Leu Leu Ile Leu Leu Gly Ala Thr Glu Ile Leu Gln Ala
      --- --- --- Met Val Pro                     Val           Leu Ala Thr

      GAC TTA CTT CCT GAT GAA AAG ATT TCA CTT CTC CCA CCT GTC AAT TTC ACC ATT AAA GTT   369
 21   Asp Leu Leu Pro Asp Glu Lys Ile Ser Leu Leu Pro Pro Val Asn Phe Thr Ile Lys Val
              Asn His Lys     Phe Leu                                            Ala

      ACT GGT TTG GCT CAA GTT CTT TTA CAA TGG AAA CCA AAT CCT GAT CAA GAG CAA AGG AAT   429
 41   Thr Gly Leu Ala Gln Val Leu Leu Gln Trp Lys Pro Asn Pro Asp Gln Glu Gln Arg Asn
                                      His     Asp                                 His

      GTT AAT CTA GAA TAT CAA GTG AAA ATA AAC GCT CCA AAA GAA GAT GAC TAT GAA ACC AGA   489
 61   Val Asn Leu Glu Tyr Gln Val Lys Ile Asn Ala Pro Lys Glu Asp Asp Tyr Glu Thr Arg
          Asp             His                     Gln         Glu     Asp

      ATC ACT GAA AGC AAA TGT GTA ACC ATC CTC CAC AAA GGC TTT TCA GCA AGT GTG CGG ACC   549
 81   Ile Thr Glu Ser Lys Cys Val Thr Ile Leu His Lys Gly Phe Ser Ala Ser Val Arg Thr
      Lys                             Pro         Glu         Ala

      ATC CTG CAG AAC GAC CAC TCA CTA CTG GCC AGC AGC TGG GCT TCT GCT GAA CTT CAT GCC   609
101   Ile Leu Gln Asn Asp His Ser Leu Leu Ala Ser Ser Trp Ala Ser Ala Glu Leu His Ala
              Lys Ser Ser     Thr Thr                 Val                     Lys

      CCA CCA GGG TCT CCT GGA ACC TCA ATT GTG AAT TTA ACT TGC ACC ACA AAC ACT ACA GAA   669
121   Pro Pro Gly Ser Pro Gly Thr Ser Ile Val Asn Leu Thr Cys Thr Thr Asn Thr Thr Glu
                                  Val Thr                         His     Val Val

      GAC AAT TAT TCA CGT TTA AGG TCA TAC CAA GTT TCC CTT CAC TGC ACC TGG CTT GTT GGC   729
141   Asp Asn Tyr Ser Arg Leu Arg Ser Tyr Gln Val Ser Leu His Cys Thr Trp Leu Val Gly
      Ser Ser His Thr His     Pro                     Arg

      ACA GAT GCC CCT GAG GAC ACG CAG TAT TTT CTC TAC TAT AGG TAT GGC TCT TGG ACT GAA   789
161   Thr Asp Ala Pro Glu Asp Thr Gln Tyr Phe Leu Tyr Tyr Arg Tyr Gly Ser Trp Thr Glu
      Lys                                             Phe     Val Leu

      GAA TGC CAA GAA TAC AGC AAA GAC ACA CTG GGG AGA AAT ATC GCA TGC TGG TTT CCC AGG   849
181   Glu Cys Gln Glu Tyr Ser Lys Asp Thr Leu Gly Arg Asn Ile Ala Cys Trp Phe Pro Arg
      Lys                         Arg     Ala     Asn     Thr

      ACT TTT ATC CTC AGC AAA GGG CGT GAC TGG CTT TCG GTG CTT GTT AAC GGC TCC AGC AAG   909
201   Thr Phe Ile Leu Ser Lys Gly Arg Asp Trp Leu Ser Val Leu Val Asn Gly Ser Ser Lys
                  Asn             Phe Glu Gln     Ala     His Ile

      CAC TCT GCT ATC AGG CCC TTT GAT CAG CTG TTT GCC CTT CAC GCC ATT GAT CAA ATA AAT   969
221   His Ser Ala Ile Arg Pro Phe Asp Gln Leu Phe Ala Leu His Ala Ile Asp Gln Ile Asn
      Arg Ala         Lys                     Ser Pro Leu                     Val

      CCT CCA CTG AAT GTC ACA GCA GAG ATT GAA GGA ACT CGT CTC TCT ATC CAA TGG GAG AAA  1029
241   Pro Pro Leu Asn Val Thr Ala Glu Ile Glu Gly Thr Arg Leu Ser Ile Gln Trp Glu Lys
              Arg         Val             Ser Asn Ser     Tyr

      CCA GTG TCT GCT TTT CCA ATC CAT TGC TTT GAT TAT GAA GTA AAA ATA CAC AAT ACA AGG  1089
261   Pro Val Ser Ala Phe Pro Ile His Cys Phe Asp Tyr Glu Val Lys Ile His Asn Thr Arg
          Leu                     Asp         Asn         Leu         Tyr         Lys

      AAT GGA TAT TTG CAG ATA GAA AAA TTG ATG ACC AAT GCA TTC ATC TCA ATA ATT GAT GAT  1149
281   Asn Gly Tyr Leu Gln Ile Glu Lys Leu Met Thr Asn Ala Phe Ile Ser Ile Ile Asp Asp
              His Ile     Lys             Ile Ala     Lys         Lys

      CTT TCT AAG TAC GAT GTT CAA GTG AGA GCA GCA GTG AGC TCC ATG TGC AGA GAG GCA GGG  1209
301   Leu Ser Lys Tyr Asp Val Gln Val Arg Ala Ala Val Ser Ser Met Cys Arg Glu Ala Gly
      Val     Thr     Ser Ile                         Pro         Met Pro

      CTC TGG AGT GAG TGG AGC CAA CCT ATT TAT GTG GGG TTC TCA AGA TAA AGGAGATAACATCCA  1272
321   Leu Trp Ser Glu Trp Ser Gln Pro Ile Tyr Val Gly Phe Ser Arg End
      Arg     Gly                             Lys Glu
```

```
      GCTTTCCTGCCCCACACCGTATCTGAAATAAAAACAACAGCAGGGATAGCAGATTAAAAAAAAAAAAAAAAAAAAAAAA  1351
```

Fig. 2

Fig. 3/1

```
                                                         CCGCTGCTTCTC  12
ATCGCATGGCCACCGCATTTCTCAGGCCAGGCACATTGAGCATTGGTCCTGTGCCTGACGCTATGCTAGATGCTGGGGT  91
TGCAGCCACGAGCATAGACACGACAGACACGGTCCTCGCCATCTTCTGTTGAGTACTGGTCGGAACAAGAGGATCGTCT  170
GTAGACAGGCTACAGATTGTTTTAGATTGAAGTTTCCTGTCATGTTCACTCATCTTTAAATCCTCATAGTAAAAAGGAT  249
```

```
    ATG ATC ATC GTG GCG CAT GTA TTA CTC ATC CTT TTG GGG GCC ACT GAG ATA CTG CAA GCT  309
1   Met Ile Ile Val Ala His Val Leu Leu Ile Leu Leu Gly Ala Thr Glu Ile Leu Gln Ala
    --- --- --- Met Val Pro                         Val         Leu Ala Thr

    GAC TTA CTT CCT GAT GAA AAG ATT TCA CTT CTC CCA CCT GTC AAT TTC ACC ATT AAA GTT  369
21  Asp Leu Leu Pro Asp Glu Lys Ile Ser Leu Leu Pro Pro Val Asn Phe Thr Ile Lys Val
            Asn His Lys     Phe Leu                                             Ala

    ACT GGT TTG GCT CAA GTT CTT TTA CAA TGG AAA CCA AAT CCT GAT CAA GAG CAA AGG AAT  429
41  Thr Gly Leu Ala Gln Val Leu Leu Gln Trp Lys Pro Asn Pro Asp Gln Glu Gln Arg Asn
                                His     Asp                                     His

    GTT AAT CTA GAA TAT CAA GTG AAA ATA AAC GCT CCA AAA GAA GAT GAC TAT GAA ACC AGA  489
61  Val Asn Leu Glu Tyr Gln Val Lys Ile Asn Ala Pro Lys Glu Asp Asp Tyr Glu Thr Arg
        Asp         His                     Gln         Glu     Asp

    ATC ACT GAA AGC AAA TGT GTA ACC ATC CTC CAC AAA GGC TTT TCA GCA AGT GTG CGG ACC  549
81  Ile Thr Glu Ser Lys Cys Val Thr Ile Leu His Lys Gly Phe Ser Ala Ser Val Arg Thr
    Lys                             Pro         Glu         Ala

    ATC CTG CAG AAC GAC CAC TCA CTA CTG GCC AGC AGC TGG GCT TCT GCT GAA CTT CAT GCC  609
101 Ile Leu Gln Asn Asp His Ser Leu Leu Ala Ser Ser Trp Ala Ser Ala Glu Leu His Ala
            Lys Ser Ser     Thr Thr                 Val             Lys

    CCA CCA GGG TCT CCT GGA ACC TCA ATT GTG AAT TTA ACT TGC ACC ACA AAC ACT ACA GAA  669
121 Pro Pro Gly Ser Pro Gly Thr Ser Ile Val Asn Leu Thr Cys Thr Thr Asn Thr Thr Glu
                                Val Thr                         His     Val Val

    GAC AAT TAT TCA CGT TTA AGG TCA TAC CAA GTT TCC CTT CAC TGC ACC TGG CTT GTT GGC  729
141 Asp Asn Tyr Ser Arg Leu Arg Ser Tyr Gln Val Ser Leu His Cys Thr Trp Leu Val Gly
    Ser Ser His Thr His     Pro                     Arg

    ACA GAT GCC CCT GAG GAC ACG CAG TAT TTT CTC TAC TAT AGG TAT GGC TCT TGG ACT GAA  789
161 Thr Asp Ala Pro Glu Asp Thr Gln Tyr Phe Leu Tyr Tyr Arg Tyr Gly Ser Trp Thr Glu
    Lys                                             Phe     Val Leu

    GAA TGC CAA GAA TAC AGC AAA GAC ACA CTG GGG AGA AAT ATC GCA TGC TGG TTT CCC AGG  849
181 Glu Cys Gln Glu Tyr Ser Lys Asp Thr Leu Gly Arg Asn Ile Ala Cys Trp Phe Pro Arg
    Lys                         Arg     Ala     Asn         Thr

    ACT TTT ATC CTC AGC AAA GGG CGT GAC TGG CTT TCG GTG CTT GTT AAC GGC TCC AGC AAG  909
201 Thr Phe Ile Leu Ser Lys Gly Arg Asp Trp Leu Ser Val Leu Val Asn Gly Ser Ser Lys
                Asn             Phe Glu Gln     Ala     His Ile

    CAC TCT GCT ATC AGG CCC TTT GAT CAG CTG TTT GCC CTT CAC GCC ATT GAT CAA ATA AAT  969
221 His Ser Ala Ile Arg Pro Phe Asp Gln Leu Phe Ala Leu His Ala Ile Asp Gln Ile Asn
    Arg Ala         Lys                         Ser Pro Leu             Val

    CCT CCA CTG AAT GTC ACA GCA GAG ATT GAA GGA ACT CGT CTC TCT ATC CAA TGG GAG AAA  1029
241 Pro Pro Leu Asn Val Thr Ala Glu Ile Glu Gly Thr Arg Leu Ser Ile Gln Trp Glu Lys
            Arg         Val             Ser Asn Ser     Tyr

    CCA GTG TCT GCT TTT CCA ATC CAT TGC TTT GAT TAT GAA GTA AAA ATA CAC AAT ACA AGG  1089
261 Pro Val Ser Ala Phe Pro Ile His Cys Phe Asp Tyr Glu Val Lys Ile His Asn Thr Arg
        Leu             Asp             Asn         Leu     Tyr         Lys
```

Fig. 3/2

```
    AAT GGA TAT TTG CAG ATA GAA AAA TTG ATG ACC AAT GCA TTC ATC TCA ATA ATT GAT GAT  1149
281 Asn Gly Tyr Leu Gln Ile Glu Lys Leu Met Thr Asn Ala Phe Ile Ser Ile Ile Asp Asp
            His Ile     Lys         Ile Ala     Lys         Lys

    CTT TCT AAG TAC GAT GTT CAA GTG AGA GCA GCA GTG AGC TCC ATG TGC AGA GAG GCA GGG  1209
301 Leu Ser Lys Tyr Asp Val Gln Val Arg Ala Ala Val Ser Ser Met Cys Arg Glu Ala Gly
    Val     Thr     Ser Ile                         Pro         Met Pro

    CTC TGG AGT GAG TGG AGC CAA CCT ATT TAT GTG GGA AAT GAT GAA CAC AAG CCC TTG AGA  1269
321 Leu Trp Ser Glu Trp Ser Gln Pro Ile Tyr Val Gly Asn Asp Glu His Lys Pro Leu Arg
    Arg     Gly                             Lys --- Arg     Ser     Val

    GAG TGG TTT GTC ATT GTG ATT ATG GCA ACC ATC TGC TTC ATC TTG TTA ATT CTC TCG CTT  1329
341 Glu Trp Phe Val Ile Val Ile Met Ala Thr Ile Cys Phe Ile Leu Leu Ile Leu Ser Leu
            His Leu         Leu Pro Thr Ala Ala     Val         Phe

    ATC TGT AAA ATA TGT CAT TTA TGG ATC AAG TTG TTT CCA CCA ATT CCA GCA CCA AAA AGT  1389
361 Ile Cys Lys Ile Cys His Leu Trp Ile Lys Leu Phe Pro Pro Ile Pro Ala Pro Lys Ser
        Arg Val             Thr Arg         Val

    AAT ATC AAA GAT CTC TTT GTA ACC ACT AAC TAT GAG AAA GCT GGG TCC AGT GAG ACG GAA  1449
381 Asn Ile Lys Asp Leu Phe Val Thr Thr Asn Tyr Glu Lys Ala Gly Ser Ser Glu Thr Glu
                    Pro     Val     Glu         Pro --- Asn         Lys

    ATT GAA GTC ATC TGT TAT ATA GAG AAG CCT GGA GTT GAG ACC CTG GAG GAT TCT GTG TTT  1509
401 Ile Glu Val Ile Cys Tyr Ile Glu Lys Pro Gly Val Glu Thr Leu Glu Asp Ser Val Phe
                Val His Cys Val     Glu Val     Phe     Val Met Gly Asn     Thr

    TGA TGACTGTCACTTTGGCATCCTCTGATGAACTCACACATGCCTCAGTGCCTCAGTGAAAATAACAGGGATGCTGGC  1587
    End

    TCTTGGCTAAGAGGTGTTCCG
```

15